Europäisches Patentamt

European Patent Office

Office européen des brevets

(11) Publication number: **0 351 516**
**A2**

## EUROPEAN PATENT APPLICATION

(21) Application number: **89108694.4**

(22) Date of filing: **13.05.89**

(51) Int. Cl.⁴: **G01N 27/28**

(30) Priority: **18.07.88 US 220246**

(43) Date of publication of application:
**24.01.90 Bulletin 90/04**

(84) Designated Contracting States:
**BE CH DE ES FR GB GR IT LI NL SE**

(71) Applicant: **PORTON INTERNATIONAL INC.**
**727 Fifteenth Street NW**
**Washington, DC 20005(US)**

(72) Inventor: **Patko, Martin J. c/o Porton**
**International Inc.**
**727 Fifteenth Street NW**
**Washington DC 20005(US)**

(74) Representative: **Hoormann, Walter, Dr.-Ing. et**
**al**
**FORRESTER & BOEHMERT**
**Widenmayerstrasse 4/I**
**D-8000 München 22(DE)**

(54) **Precalibrated, disposable, electrochemical sensors.**

(57) Electrochemical sensors that are disposable,
precalibrated and capable of measuring a single or
multiple parameters are disclosed. The sensors can
be mass produced and are designed to be operated
by personnel with a minimum of skills. The sensors
are packaged in substantially water and oxygen im-
permeable containers and are particularly designed
for use in the medical field to measure various ions
or other species in body fluids.

Fig.2

## PRECALIBRATED, DISPOSABLE, ELECTROCHEMICAL SENSORS

### BACKGROUND OF THE INVENTION

Laboratory data should be available rapidly and preferably continuously when a physician is caring for a critically ill patient. It is frequently necessary to analyze blood for electrolytes such as potassium, sodium, calcium, chloride and other species as rapidly as possible. The ability to measure ionized potassium, for example, to rapidly provide real time data to the physician is frequently of critical importance. The gradient of potassium across the cell membrane is the principal contributor to the membrane potential. Maintenance of this potential is essential for the normal function of all nervous and muscular tissue, including the conducting and contracting elements of the heart. The rapid measurement of potassium ion is important in intensive care, the administration of digitalis, and in the treatment of burn patients and diabetic patents.

### SUMMARY OF THE PRIOR ART

U.S. Patent 4,340,457 to Kater describes an ion selective electrode capable of in vivo monitoring.

U.S. Patent 3,700,576 to Bloch et al, describes an ion activity measuring electrode.

U.S. Patent 4,293,307 to Simpson et al, describes a method and apparatus for analyzing a liquid such as blood for potassium ions, calcium ions or dissolved gases.

U.S. Patent 4,508,613 to Busta et al, describes a miniaturized potassium ion sensor.

U.S. Patent 4,639,424 to Wong describes a method for determining the alkali metal content of protein containing liquids.

### BROAD DESCRIPTION OF THE INVENTION

The electrochemical sensors of the instant invention will be described as several basic arrangements.

The monoprobe arrangement comprises an electrochemical sensor measuring a single parameter with or without temperature compensation.

The second basic arrangement is the multiprobe best defined as a complex electrochemical sensor measuring multiple parameters with or without temperature compensation.

The third basic arrangement is the microprobe. In this arrangement the design for both mono and multiprobes are advanced to a microprobe.

Three types of sensing electrodes maybe utilized in the disposable sensors. The first type is glass membrane based. This type of sensing electrode typically utilizes a flat glass membrane of known configuration for pH, sodium, potassium, caesium, lithium and other ions.

The second type of sensing electrodes are solid membrane based. Solid membrane electrodes are mainly characterized as ion selective electrodes.

The third type of sensing electrodes are organic membrane based. Although there are a large variety of organic polymer based sensing electrodes they all have common properties.

In addition there are sensing electrodes based on secondary reactions. There are many enzymes, hormones, antibiotics and other compounds that, if in contact with a particular sample, produce or consume species that are measurable with electrochemical sensors.

Gas sensors are a sensor variation representing a sensing electrode with a secondary reaction.

### DETAILED DESCRIPTION OF THE INVENTION

For purposes of simplicity the various arrangements will be discussed individually.

The monoprobe arrangement consists of up to six basic part;

    a. A sensing electrode,
    b. A reference electrode,
    c. A temperature compensator,
    d. Shielding,
    e. A data collection device, and/or
    f. A connector system.

The sensing electrode is located typically in the center of the sensor. It is preferably circular in shape and is separated from the reference electrode by an electrical insulator. The insulation has to be three orders of magnitude higher in resistance than the resistance of the sensing electrode if an accuracy of 0.1% is required. However, if an accuracy of 1% is required a resistance of two orders of magnitude is sufficient.

The reference electrode typically encloses the sensing electrode, serving two basic functions. It provides a reference electrode potential and provides a shield for the sensing electrode against static and magnetic fields. The sensing electrode is either indented relative to the reference electrode or at the same level to assure coverage of the sensing electrode by the sample.

The temperature compensator increases the accuracy of the measurement where the calibrating temperature and the temperature of the sample may differ. In selecting a temperature compensator the circuit design and the available space in the sensor are the controlling considerations.

The shield is a metallic or plastic conductor that surrounds the measuring electrode and/or maybe a mesh system based on the principles of the Faraday cage. The shield must also be insulated from the sensing electrode and the calibrating system. In certain instances the shield may also serve as a moisture and gas barrier.

A data collection system is built into the disposable sensor. This device is conductive and can carry AC and DC signals, and is inert and passive to the sensing and reference electrodes. A connector system provides the communication with the instrument.

The second basic arrangement is the multiprobe system. The multiprobe is a complex electrochemical sensor measuring multiple parameters with a thickness less than the diameter of the average axis of the sensor. The multiprobe has the same six basic parts as the monoprobe, with the following differences.

The reference electrode(s) are typically arranged in the center of the sensor with the sensing electrodes surrounding the reference electrode(s).

The description of the insulation, temperature compensator, shield and data collection device relating to the monoprobe set out above apply to the multiprobe as well.

The third basic arrangement is the microprobe.

The necessary design change over monoprobe and multiprobe arrangements is the addition of a cover on top of the sensor having channels in it connecting the sensing and reference electrodes by a narrow gap, but exposing the whole active surface of the electrodes. This arrangement greatly reduces the amount of sample required to make the measurement.

The advantage is more significant in case of a multiparamater measurement, where only the channel and electrode surfaces have to be covered with the sample.

Packaging is another basic feature of the sensors. The sensors are packaged in substantially water, carbon dioxide and oxygen; impermeable containers. A typical container is composed of;

a. Multilayer preformed plastic embracing the shape of the electrode,

b. Single layer plastic packaging surrounding the electrode,

c. A solid plastic block formed in the shape of the electrode, or

d. A half shell covering the top portion of the electrode, and/or

e. A metal coated plastic in versions a through d. However in version d the outer surface of the sensor may be metallized.

The packaging protects the solvents of the immobilized reference electrode filling solution and the organic components from evaporation and oxidation. The packaging also eliminates concentration change of the calibrating immobilized filing solution, provides mechanical strength and durability during storage and transportation, assures the required shelf life and provides an electrical shield against static, magnetic and electrical fields.

The sensing electrodes may be glass membrane based, solid membrane based or organic membrane based.

The glass membrane typically utilizes a flat glass membrane of known configurations for pH, sodium, potassium cesium, lithium and other ions.

The novelty of these electrodes is in the internal design of the filling and reference half cell system;

1. utilizing immobilized filling solution without an air gap, with a sealing layer in immediate contact with the immobilized filling solution, and topped with one or two layers of additional sealant, where the immobilized component may be:

a. inorganic based gel

b. organic based gel or a

c. polymerized macromolecular gel.

The gel is polymerized from a water soluble or water unsoluble monomer. The immobilized component may also be a cross linked polymer wherein the cross linking is chemically initiated, light initiated on gamma ray initiated.

All these filling solutions contain the required ionic components to provide the necessary pH value, ionic composition and ionic strength and osmostic pressure to assure long range stability for the filling solution and the electrode. Coloring agents for easy identification purposes and preservatives may be added.

In addition, a solid state internal reference system for glass-based membrane electrodes consisting of a salt with a melting temperature at about the transformation temperature of the glass membrane, where one ion of the salt is able to penetrate the glass structure and can be captured at room temperature, may be used. A solid conductor provides electrical contact with the solid state filling solution. These types of electrodes can be made extremely "thin" with large diameter to height ratio.

The glass membrane may also be a lens-like sensing surface with vacuum deposited conductive contacts or solid contacts with adhesives incorporating conductive materials.

The solid membrane based sensing electrodes are mainly characterized as ion selective.

A conductive substrate with approximately the same thermal expansion coefficient as the solid membrane in question can be used to vacuum deposit, sputter or using other thick membrane technology to produce the sensing element of the electrode. These substrates can be made in a way that they contain the metal, the ion, or both which the membrane deposited onto is reversible or conductive for. These membranes can be mass produced for low cost and can be mass handled for preparations, like mechanical or electrochemical polishing and can possess a mechanical design suitable for automated assembly, such as using robots.

The sensing electrode can be based on an organic membrane.

Although there are a large variety of organic polymer based sensing electrodes, there are some common properties that make it feasible to design disposable precalibrated sensors. The base polymer providing the matrix for the membrane must be soluble in a solvent or solvent mixture; a suitable plastic must be found to solvent bond the membrane to the plastic; the plastic must be an electrical insulator; the membrane must have a reasonably high chemical resistance against water and other solvents. Immobilizing agents and polymers used in the sensor can provide the required shelf life and lifetime for the sensing electrode; the membrane must have an acceptable mechanical strength with or without support or substrate to withstand the handling and measuring procedures.

The membrane must have one of the following configurations:

(a) The membrane is installed on a reference electrode substrate where the membrane is in direct contact with a solid substrate which contains a reference electrode system with or without an immobilized filling solution. This substrate is typically surrounded by the plastic insulator onto which the membrane is solvent bonded. For additional mechanical strength, in the center of the substrate, there may be an additional plastic disk, Thus the membrane forms a donut shape. In the case of a multiprobe arrangement this center plastic may contain one of the reference electrodes.

(b) The membrane installed on a salt bridge not containing immobilized filling solution. In this case the membrane is typically solvent bonded to the plastic which incorporates a porous area of either plastic or other material to which the membrane reasonably adheres. The sensing electrode is backed with a reference electrode in contact with an immobilized filling solution.

(c) The membrane installed on a salt bridge, salt bridge saturated with immoblized filling solution. The sensing electrode is backed up with a reference electrode in contact with an immobilized filling solution.

The sensing electrodes may be based on secondary reactions. There are many enzymes, hormones, antibiotics, antibodies and other compounds that, if in contact with a particular sample, produce or consume species that are measurable with electrochemical sensors. These compounds need to be impregnated into a membrane covering the sensing electrode, and not to be part of the calibrating system but containing the same immoblized calibrating system as the main immobilized calibrating system. After removal of the main immobilized calibrating system a known amount of the calibrating agent remains in the impregnated membrane. If the secondary reaction induced by the sample with the special compound produces or consumes the calibrating agent, the sensor will be able to indicate the fact through the change of the electrode potential. This change in the electrode potential can be processed so as to represent the concentration or activity of the sample.

The sensor may be as a gas sensor. The gas sensor is a sensor variation representing a sensing electrode with a secondary reaction. If the impregnated membrane contains a compound which through the reaction with the gas induces a change in the compound the sensing electrode becomes a gas sensor, which can be produced without the use of a known gas permeable membrane. The traditional gas sensors also can be manufactured in a precalibrated, disposable configuration if, on top of the impregnated membrane, an additional gas permeable membrane is used.

The reference electrode has the following characteristics. The role of the reference electrode in electrochemical measurements is to provide a stable, reproducible reference potential for the measurement which is essentially independent of the composition and concentration of the sample. This is true to the extent that, because of the composition difference of the sample and of the filling solution of the reference electrode, there always will be a small, so called diffusion potential. The intention is to keep this potential at the minimum level possible. The role of the reference electrode in the disposable precalibrated electrochemical sensor is very important. However, it needs to provide a stable and reproducible potential only twice once during the time the instrument is "picking up" the information from the precalibration, and the second time, when the actual measurement with the sample takes place. The other requirements, the elimination of the diffusion "junction" potential during the measurement, takes the higher priority. The requirements will be satisfied by the use of an immobilized reference electrode filling solution, which needs to be composed for the possible minimum of junction potential.

The immoblized filling solutions for the reference electrodes must satisfy the following:

(a) The immobilization keeps the reference electrode filling solution in place from the time of manufacture to the time the measurement is completed and the sensor is disposed.

(b) The immobilization must contain the compound to produce a stable reference electrode potential during the lifetime of the sensor.

(c) The immobilization must assure through composition, ionic strength and osmotic pressure that the minimum diffusion "junction" potential will develop during the measurement.

(d) The immobilized reference electrode filling solution must be inert and indifferent to the calibrating solution, and must not alter the calibrating solution during the shelf life of the electrode. This requirement can be satisfied in most cases by using exactly the same immobilized filling solution as the calibrating solution or by devising a calibrating solution to satisfy the reference electrode filling solution requirements.

The immobilization of sensing electrode filling solutions is essential in providing a feature which permits the sensor to be stored, transported and used in any position. This immobilized solution has to be of an appropriate composition to provide a stable potential for the sensing membrane and for the internal reference electrode. If the sensing membrane is permeable to any of the components of the immobilized filling solution of the sensing electrode or to the immobilized calibrating solution, the same considerations must be met as in the case of the immoblized filling solutions for reference electrodes.

If there is a salt bridge for certain types of designs, either on the reference electrode side of the sensor, or on the sensing electrode side of the sensor, the same basic requirements apply as for the immoblized filling solutions for references electrodes, or the immobilized filling solutions for sensing electrodes, respectively. In addition to these requirements, the immobilized filling solution for salt bridges have to be inert and indifferent to the material selected for the salt bridges.

For applications where the sample temperature is different than that of the environment, and the measurement has to be taken at that temperature, a temperature compensator is usually necessary to achieve a high degree of accuracy. This is a temperature indicator connected to the instrument allowing the instrument to use a known temperature coefficient to correct the value to the actual temperature, if the "calibration" was carried out at a different temperature than that of the measurement, or to display a value corrected to any chosen so called "standard" temperature. The temperature compensator can be very different in nature, from resistors to semiconductors, until the parameter measured by the change of temperature is reproducible and the sensitivity is adequate for the chosen application. For the disposable sensor the smallest possible size is advantageous because of the limited space available. It is preferably located in between the sensing and reference electrode. Its response time should be commensurate to that of the sensor. The temperature sensor typically needs two connectors. One of the connection points may be joined with the reference electrode connection. If the sample is allowed to equilibrate with the sensor and the environment, and the only temperature change is coming from the environment of the testing, a temperature compensator may be built into the instrument. In this case the "calibration" of the instrument may be done at one location at one temperature and the measurement will be permitted to be performed at a different location at different temperature, or at a different temperature at the same location.

Electrochemical sensors are of high resistance in nature and for that reason susceptible to interferences from static electric fields, high voltage electric fields, magnetic fields and capacitance change in their vicinity. This interference may be indicated as a noise, unstable reading or in case of auto-read systems, a long response time and nonrepeatable measurement. In the event an extrapolating or predictor circuitry is involved it may completely disfunction the instrument or result in a nonreproducible reading.

For all the purposes listed above proper shielding is necessary for the disposable, precalibrated electrochemical sensor.

This can be achieved three different ways. These approaches may be combined or used independently.

The reference electrodes, if encircling the sensing electrodes, acts as a first line shielding. In addition, conductive plastic or metallic components may be built into the sensor housing completely covering the cross section of the sensor in a three dimensional way. The third solution to the problem is metal or conductive plastic coated packaging. Both of the latter versions have to be properly insulated from the sensing electrode and tied electrically to the reference electrode or to instrument ground depending on the design of the instrument.

Calibration and recalibration are the most time-consuming and error prone procedures in the application of electrochemical sensor. These disadvantages can be overcome by using precalibrated, disposable sensors. In this case the sensor is in contact with the calibration system from the time of manufacture and is always ready for use. The calibration information is inherent in the sensor packaging, and can be "picked up" when connected to an instrument designed for the use of such a sensor. Thus, no time or effort is required for calibration and potential operator error is prevented. Thus, the sensor can not be recalibrated, eliminating recalibration error.

The immobilized calibrating system contains complex chemistry to accommodate the design requirements and to accommodate the requirements of precalibration. The components of the immobilized calibrating system are as follows:

A. Immobilizing agents. These agents are typically a multicomponent system produced in single or multiple step operations. For precalibration purposes they are preferably immobilized from liquid phase in the assigned space in the sensor itself. After immobilization the system is typically of high viscosity or of solid consistency. It follows the shape of the sensor to stay in contact with the electrodes. The system is designed not to change consistency considerably during the shelf life of the sensor. A partial list of candidates which may be used as immobilizing agents includes:

a. inorganic based gel;

b. organic based gel;

c. polymerized macromolecular gel, polymerized from water soluble or insoluble monomer;

d. crosslinked polymer, chemically initiated crosslinking;

e. crosslinked polymer, light initiated crosslinking;

f. crosslinked polymer, particle or gamma ray initiated crosslinking;

g. crosslinked organic gel, chemically initiated crosslinking;

h. crosslinked organic gel, light initiated crosslinking; or

i. crosslinked organic gel, particle or gamma ray initiated crosslinking.

The major factor in preserving the concentration of the calibrating agent is to control the vapor pressure of the immoblized calibrating system. The internal vapor pressure and the gradient to the outside of the package are the major driving forces to loose solvents. This results in concentration changes in the system. Some additional agents may be included in the composition of the system to balance these forces. There are many such agents to choose from; examples are di-and trivalent alcohols, poly-alcohols etc.

The calibrating agent is unique to each sensing electrode and tailored in concentration or activity to the sample. Both the immobilizing and vapor pressure controlling agents must be compatible with the calibrating agent.

The ionic strength governs the osmotic pressure of the system, the activity of the calibrating agent, the junction potential of the reference electrode and may influence the electrochemical selectivity of the sensor toward the sample. The components to be chosen for the ionic strength adjustment have to take all of the above factors into account.

The immobilized calibrating system needs to be removed from the sensor after the calibration information is "picked-up" by the appropriate instrumentation. In order to support this removal the inclusion of the physical barrier is advantageous to separate the sensing and reference electrodes and other sensor components form the immoblized calibrating system. This barrier, or separator, must be permeable to the calibrating agent, allowing it to stay in contact with the sensing and reference electrodes. The separator may be completely removed, or only the portion covering the sensing or the reference electrodes be removed.

Supporting components for the above systems may be added that do not interfere with any of the other components. Such items may be: stabilizers for macromolecules, u.v. absorbers, preservatives or coloring agents. The chemical balance of the entire system must be maintained if these types of additives are incorporated.

To insure the longest possible shelf life of the sensor, the original conditions of the complete precalibrated, disposable sensor must be preserved. Preservatives to perform this function may be added to any of the components. Basic requirements for the preservatives are: to be inert, inactive, non-poisonous to the sensing and reference electrodes, and ideally not containing any components of the sensor itself. If this factor is unavoidable, the presence of the preservative has to be taken into account when the others are formulated.

If it is required for any application that the precalibrated, disposable sensor has to be completely sterile it may be designed to accommodate that requirement. Gamma ray sterilization will sterilize the interior and the exterior of all electrodes and components of the sensor. There are some material requirements to be fulfilled in the design of such a sensor: all materials selected have to be resistant to gamma radiation, or the levels of degradation and recombination (especially in plastic materials) must be approximately at the same level. For the integrity of the packaging of the immobilized calibrating system no component should release any gases or components volatile at low temperature.

If gas sterilization is required for any application it can easily be achieved by selecting a packaging material permeable to the sterilizing gas. The gas sterilization will not sterilize the interior of the electrodes, but will sterilize the exterior and the immobilized calibrating system. In general, the same basic requirements will apply as in case of the preservatives to maintain the integrity of the sensor.

If chemical sterilization is required, a set of preservatives have to be selected to satisfy the requirement. The sterilization of the electrode surfaces prior to measurement is, in most instances, not feasible in most common cases.

The connectors service multiple purposes. They completely or partially hold in place the sensor in or on the instrument. They serve as a recognition device for the instrument to verify the existance, proper connection and the type of sensor to the instrument. The other function is to collect information about the condition of the sensor before the calibration information is taken from the sensor. The last function is to transmit the calibration information and all information about the measurement with the sensor.

The connectors and the electrodes attached to them may not be adequate to supply all the necessary information to the instrument to assure a trouble and operator assistance free operation. The most information can be collected, if independent data collection systems are established. These in concern with the instrument provide all the necessary information about the condition of the sensor. The systems are conductors, plastic or metallic, located in the vicinity of the electrodes. They are able to transmit signals from the instrument, and collect responses and data for the analysis by the instrument about the readiness of the sensor to fill its functions. The conductor have to be inert chemically and inactive to protect the integrity of the sensor. Separate connections to the instrument are necessary to service their purposes.

An instruments must have the following items and features to be able to accommodate a precalibrated, disposable sensor.

a. The instrument has to be capable to generate and analyze AC and DC signals to obtain information about the condition and readiness of the sensor.

b. The instruments has to be capable to collect AC sinus and non sinus form voltage and current data and DC potential data. If equipped with a bar code reader, mechanical or electronic coding devices such as microchips and the like, the device can then provide means which read a bar code providing serial number of sensor, expiration date of sensor, type of sensor, polarity of sensor, initial slope, slope change information, calibration point, potential ranges at calibration points, response time limits.

c. The instrument has to assure through data processing and analysis the reliability of the information collected about the condition of the sensor via the calibration and measurement process. It has to convert obtained information into the same form of information that is already permanently stored or is temporarily obtained from the sensor before and after connection and just temporarily stored in the memory for comparison and analysis purposes. The information which is time - dependent need to be filtered through either auto-read or predictor circuitry.

d. Data are preferably displayed in digital form in any chosen concentration or other units either on an electronic display device or in printed form. Wireless transmission or collection in the instrument itself for central storage and statistical analysis purposes is an option and preferable addition to the capability of the instrument.

BRIEF DESCRIPTION OF THE DRAWING

The invention is illustrated by the following drawings.

Figure 1 is a perspective view of one embodiment of the packaged disposable electrochemical sensors

Figure 2 is cross sectional view of package taken along line 2-2 of figure 1.

Figures 3 is a top view of package taken along line 3-3 of figure 2.

Figure 4 is a perspective view if the sensor with the protective cover removed showing the method of preparing the electrode for use.

Figure 5 is a perspective view showing the slant sensor for analyzing blood.

Figure 6 is a top view of the sensor of figure 5.

Figure 7 is a cross sectional view of the slant sensor with a blood sample in place.

Figure 8 is a top view of the sensor showing the multiprobe arrangement

Figure 9 is a cross sectional view taken along line 9-9 of figure 8.

Figure 10 is an exploded view of the area 10 of figure 9.

Figure 11 is a cross sectional view of the devie especially designed for measuring potassium.

Figure 12 is a top view of the device of figure 11.

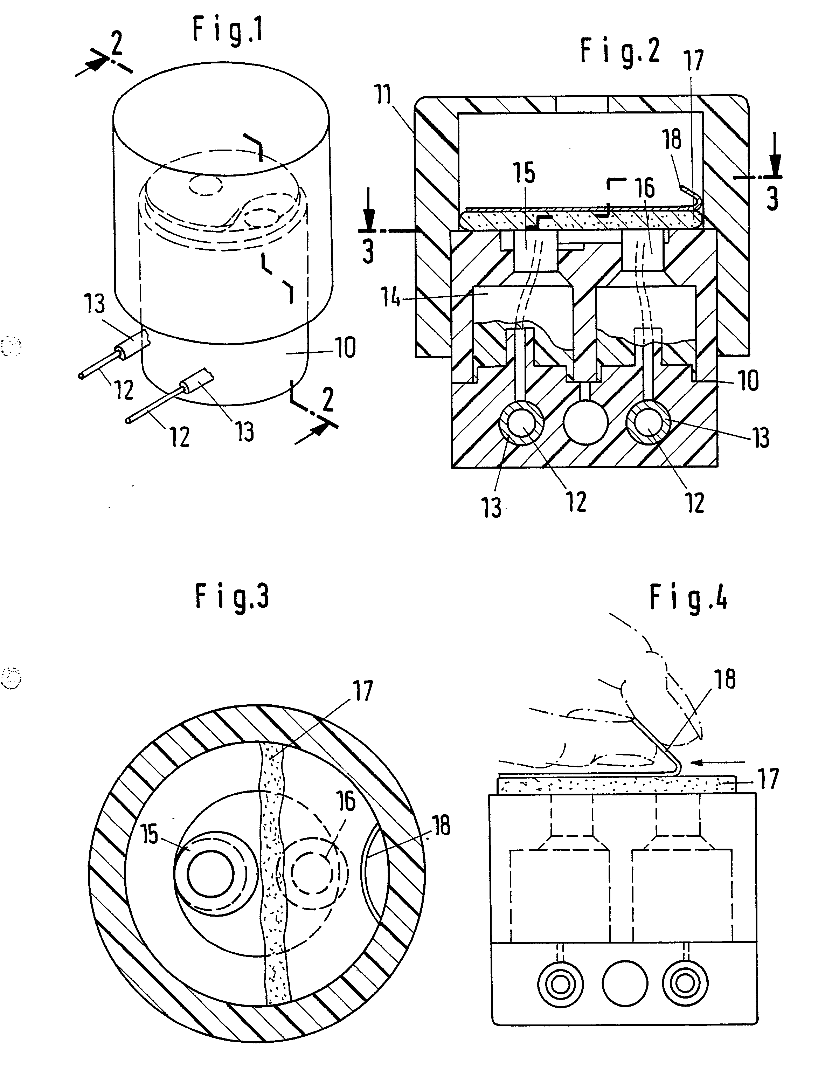

Referring now to figure 1, which shows the disposable electrochemical sensor package for delivery to the user. The ensor 10 is covered with a plastic cap 11 that fits over the sensor connecting lines 12 protrude from the base of the sensor through insulators 13.

Figures 2 is a cross sectional view of the package showing the plastic over 11, a monoprobe 14 consisting of a sensing electrode 15 and a reference electrode 16. The monoprobe is covered by a sponge like material 17 that in turn is covered by a peelable plastic over 18. The figure also gives a cross sectional view of the connecting lines 12 surrounded by the insulators 13.

Figure 3 is a top view of the package of figure 1 showing the sensing electrode 15 and the reference electrode 16 with the sponge like material 17 and the peelable cover 18.

Figure 4 shows the method of removing the peelable cover 18 from the sponge like material 17.

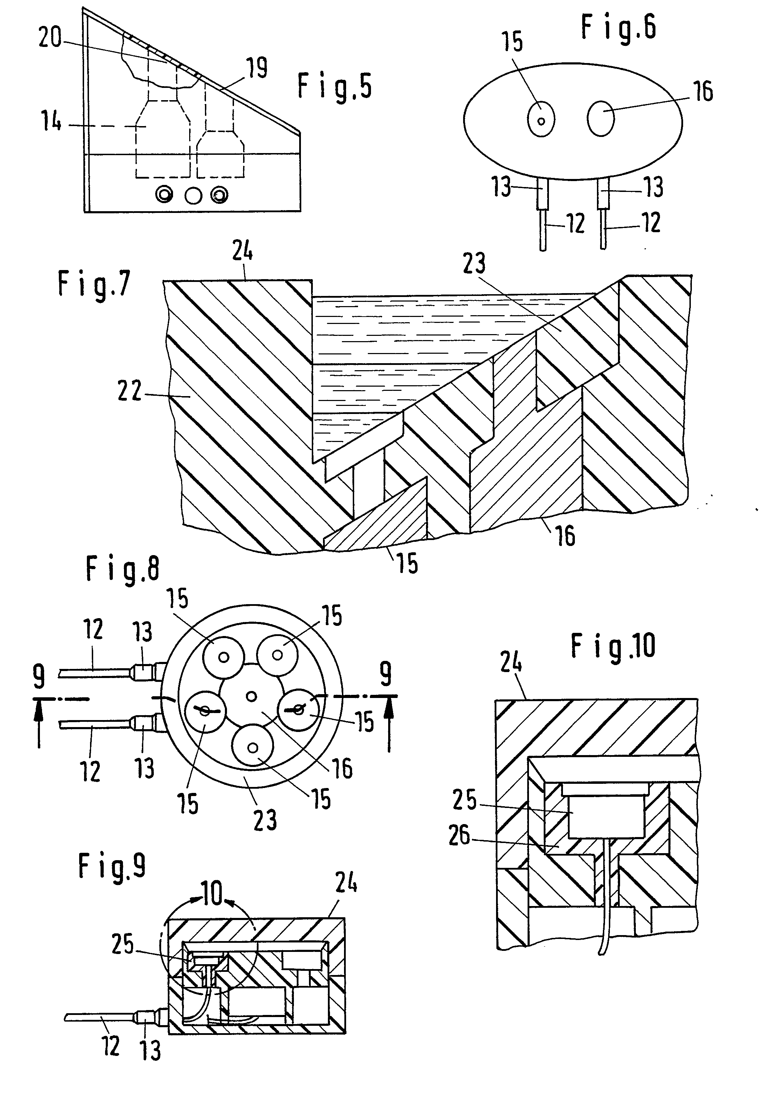

Figure 5 shows a monoprobe 14 having a slant top 19. This embodiment is designed to collect blood samples directly from the patient by advancing the painted member 20 to draw blood from the patient directly onto the top 19.

Figure 6 is a top view of the monoprobe of figure 5 showing the position of the reference electrode 16 the sensing electrode 15., the connecting lines 12 and the insulators 13.

Figure 7 is a cross sectional view of another embodiment of the invention. In this embodiment the plastic container 22 surrounds the slant top 23 covering the sensing electrode 15 and the reference electrode 16. The fluid to be tested 24 is shown in the plastic container 22.

Figure 8 is a top view of the sensor with a multiprobe arrangement. The device includes the plastic case 23, and 5 sensing electrodes 15 and a reference electrode 16. The connections 12 are shown surrounded by the insulation 14.

Figure 9 is a cross sectional view showing a microprobe package. The device includes a plastic cap 24, the microprobe, the connector 12 surrounded by the insulation 13.

Figure 10 is an exploded view of the area 10 in figure 9 showing a portion of the cover 24, the microprobe surrounded 25 by plastic insulation 26 and showing a portion of the connector 12.

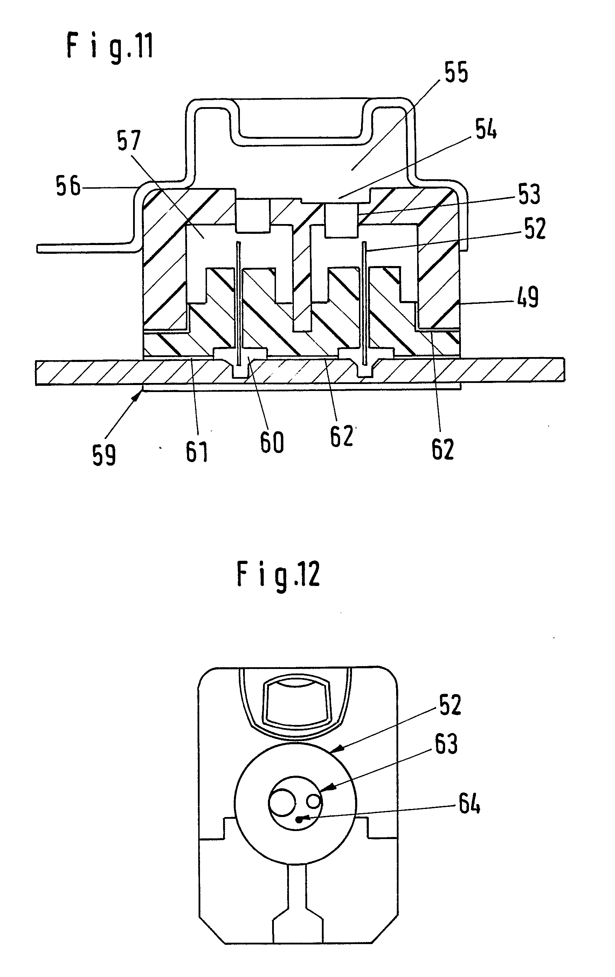

In the device designed to measure potassium shown figure 11 the device 49 includes the electrodes base 50 the electrodes body 51 the half cell wire 52, the ceramic junction 53, the membrane 54, the calibration gel 55, the peel away cap 56, and the immobilized gel 57. The substrate card is shown at 58 with the bar code label shown at 59. A layer of conductive epoxy resin is shown at 60 and a layer of non conductive resin is shown at 61. The elements are jointed by ultrasonic welds shown at 62.

Referring now to figure 12 which is a top view of the device of figure 11 showing the electrode body 51 and the sample cavity 63 and the electrode 64.

The function of each of the elements shown in figures 11 and 12 is described below.

The electrode base 49. This injection molded PVC part, holds the two Ag/AgCl reference half cells inserted into it and serves at the main electric insulator inbetween the potassium sensor and the reference electrode electrode. This part connects the electrode body to the substrate card (connector) and seals the reference half cell cavities when welded to the electrode body.

The electrode body 51. This injection molded PVC part is the main assembly of the sensor. It contains the two reference electrodes, one for the potassium sensor and one for reference purposes. The two porouos junctions provide (Ionic) connection to the calibrating gel and to the potassium membrane.

The half cell wire 52 consists of two silver wires chloridized on the portion in contact with the immobilized gel build the reference half cells.

The ceramic junction 53 consists of two monolytic ceramic plugs with high porosity saturated with the immobilized reference gel.

The calibration gel 55 permits the design of a sensor and instrument which does not require either a sophisticated calibration process or a well educated operator. It keeps the sensor always in a calibrated state and ready for the instrument to pick up the calibration value. It's composition is exactly the same as that of the immobilized reference gel. The calibration and immobilized reference gel can be gamma ray sterilized up to 7,5Mrad. The gamma ray induced degradation and recombination rates are approximately the same therefore there is no change in the consistency and composition of the gel.

The bar code label 59 contains information for process and calibration of the sensor. The date code will instruct the user not to process sensors beyond the expiration date. The additional information such as initial slope of the potassium sensor will increase the precision of calibration and enhance the accuracy of the measurement; the information containing the change of slope by time will extend the usable life time while maintaining the initial accuracy of the sensor.

The non-conductive epoxy 61 serves to bond the electrode base to the substrate and insulates the half cell wires from each other.

The conductive epoxy 60 provides electric contact from the half cell wires to the conductors on the substrate.

The potassium membrane 54 is the signal producing part of the sensor. It is a multicomponent plasticised PVC membrane which is solvent bonded to the electrode body.

The substrate card 53 is an injection molded plastic or prefabricated ceramic part. The metallic conductors are electroplated onto the substrate in a separate process (see figure 12) The electric connection to the reference half cells is provided by a conductive epoxy.

The immobilized gel 57 is an immobilized polymeric gel crosslinked from a water soluable polymer. It contains the ions assuring a stable reference potential for both potassium and reference electrodes and the ionic activity to calibrate the potassium sensor, for the long term stability of the sensor and for the elimination of the driving forces of ion migration (concentration gradients, osmotic pressure differences). Its composition is the same as that of the calibrating gel.

The peel-away cap 56 is a multilayer polymeric foil practically impermeable to water vapor, either vacuum formed or deep drawn to the designed shape. It is welded to the electrode body and contains the calibration gel. After completion of the calibration process in combination with the instrument it will be peeled off to expose the potassium sensor and the reference electrode to the sample (blood) filled into the sample chamber above the potassium membrane and ceramic junction.

There is a component not visible on the cross section of the sensor, it is a third electrode consisting of a silver wire located in the sample chamber and connected to the third conductor of the substrate on the top view in figure 12. It's function is noise reduction utilizing a differential amplifier in the instrument.

Obviously, many modifications and variations in the invention may be made without departing from the essence and scope thereof, and only such limitations should be applied as are indicated in the appended claims.

## Claims

1. A system for rapidly determining the concentration of a component in body fluids which comprises exposing a sample of said body fluids to a precalibrated disposable electrochemical sensor.

2. The precalibrated, disposable electrochemical sensors of claim 1 wherein the basic probe arrangement consists of a monoprobe, a multiprobe or a microprobe consisting of up to six basic elements.

3. The basic probe arrangement of claim 2 wherein said elements consist essentially of a sensing electrode, a reference electrode, a temperature compensator, shielding, a data collecting device and a connector system.

4. The basic probe arrangement according to claim 2 wherein the sensing electrode or electrodes, and the reference electrode or electrodes are positioned in the center of the sensor and are separated by an electrical insulator.

5. The basic probe arrangement according to claim 2 wherein the shielding is a metallic or plastic conductor surrounding the measuring electrodes.

6. The basic probe arrangement according to claim 2 wherein the sensing electrodes comprises glass membranes which incorporates a reference half cell and a filling solution and a reference half cell that utilize an immobilized solution covered with one or two layers of sealant.

7. The monoprobe, multiprobe and microprobes according to claim 6 wherein said immobilized filling solution is selected from the group consisting of an inorganic based gel, and organic based gel a polymerized macromolecular gel and a cross linked polymer.

8. The basic probe arrangement according to claim 2 wherein the sensing electrodes selected from the group consisting of ion selective solid membranes, organic membranes, electrodes based on secondary reactions and gas sensors.

9. The sensing electrodes according to claim 8 wherein enzymes, hormones or antibiotics are impregnated into a membrane and cause a secondary reaction that produce or consume species and the reaction is measured with the electrochemical sensors.

10. The precalibrated disposable electrochemical sensors according to claim 2 which also include vapor pressure control agents, calibrating agents, ionic strength adjustors and preservatives.

11. The sensory electrodes of claim 9 wherein a bar code and bar code reader are included as a signal reading means.

12. The sensory electrodes of claim 11 wherein the bar code defines a serial number of a sensor, an expiration date of a sensor, a type of a sensor, a polarity of a sensor, slope information of a sensor, calibration point of a sensor, potential range of a sensor, response time limits of a sensor; and the like.

Fig.1

Fig.2

13

12

12

13

10

2

2

11

17

18

15

16

14

10

13

13

12

12

Fig.3

Fig.4

17

15

16

18

18

17

Fig.5

Fig.6

Fig.7

Fig.8

Fig.9

Fig.10